# EUROPEAN PATENT APPLICATION

(11) **EP 2 228 066 A1**
(43) Date of publication of application: **15.09.2010**
(21) Application number: 09154212.6
(22) Date of filing: 03.03.2009
(51) Int. Cl.: A61K 31/64, A61K 31/198, A61K 31/155, A61K 47/18, A61K 9/16, A61K 9/20, A61P 3/10

(54) **Pharmaceutical compositions of sulphonylurea-based active pharmaceutical ingredient with excellent dissolution properties**

(71) Applicant: LEK Pharmaceuticals d.d., 1526 Ljubljana (SI)
(72) Inventor: The designation of the inventor has not yet been filed

(57) **Abstract**

The present invention provides a pharmaceutical composition comprising at least one sulphonylurea-based active pharmaceutical ingredient, wherein the at least one sulphonylurea-based active pharmaceutical ingredient is intimately mixed with a basic agent. The weight ratio of the basic agent and the at least one sulphonylurea-based active pharmaceutical ingredient is from above 1:1 to 50:1, especially from 1.1:1 to 50:1, preferably from 1.5:1 to 25:1, more preferably from 2:1 to 15:1, yet more preferably from 3:1 to 10:1. The pharmaceutical composition can be obtained by simply mixing sulphonylurea-based active pharmaceutical ingredient and the basic agent in the aforementioned weight ratio, optionally in the presence of the granulation liquid. Alternatively, the pharmaceutical composition can be obtained by at least partially or fully dissolving the compounds in the granulation liquid or solvent, and at least partly precipitating them. The invention provides improved dissolution properties of the sulphonylurea-based active pharmaceutical ingredient.

## Description

### Field of the invention

The present invention relates to pharmaceutical composition comprising at least one sulphonylurea-based active pharmaceutical ingredient being mixed with a basic agent. Further, the present invention provides for a process and use of said pharmaceutical composition.

### Background of the invention

Sulphonylurea-based active pharmaceutical ingredients have proved to be a class of potent drugs for treating diabetes. Sulphonylurea-based active pharmaceutical ingredient lowers blood glucose primarily by stimulating the secretion of insulin from functional pancreatic beta cells. In this way they exert a long-term effect of reducing the blood glucose levels. In addition, extrapancreatic effects may also play a role in the activity of sulfonylureas. A particular sulphonylurea-based active pharmaceutical ingredient is glimepiride. This is supported by both preclinical and clinical studies demonstrating that glimepiride administration can lead to increased sensitivity of peripheral tissues to insulin. However, the characteristic low solubility of sulphonylurea-based active pharmaceutical ingredients poses great difficulty to providing pharmaceutical composition of suitable solubility or dissolution rate. In such case, when active pharmaceutical ingredient is not soluble or only sparingly soluble in neutral or acidic pH region, the dissolution is poor and a sufficient blood concentration of active pharmaceutical ingredient can not be properly obtained. Specifically for the sulphonylurea-based active pharmaceutical ingredients the prior art describes various solutions to mitigate low solubility problem.

US 2004/0147564 A1 discloses a pharmaceutical composition for oral administration comprising glimepiride which has a mean particle size of less than about 30 microns and a particle size distribution such that at least 90 % glimepiride particles are less than about 75 microns.

In the US 2007/0264331 A1 immediate release of glimepiride was achieved by putting glimepiride in a film coating, wherein sodium lauryl sulphate was added. In addition, the glimepiride was micronized.

A rapid release portion comprising sulphonylurea-based antidiabetic medicine as an active ingredient was disclosed in WO 2006/071078. The rapid release portion was coated on a controlled release portion and further comprised a stabilizer for the active ingredient, wherein the stabilizer was selected from the group of an antioxidant, an inorganic base, an organic base and a basic amino acid and the weight ratio of the stabilizer and sulphonylurea-based antidiabetic medicine ranged from 0.01:1 to 1:1.

US 6,693,094 B2 describes a unit dosage form comprising sulphonylurea or metformin and a number of further active agents in a concept to reduce the risk of insulin resistance and type 2 diabetes. Active agents other than sulphonylurea or metformin include a range of substances categorized into different metabolic activities, i.e. compounds occurring naturally within the body like, among many others, arginine. The amounts of sulphonylurea and arginine were from 1 mg to 20 mg and from 75 mg to 3100 mg, respectively.

Generally, as a method of improving dissolution property of sparingly soluble drug, several methods such as obtaining micronized particles of active pharmaceutical ingredient by pulverization or spray dry method, even nanosizing, have been known. In case of reducing particle size special equipment or a complicated operation processes are required for the production. Conventional methods of particle size reduction rely upon mechanical stress to disaggregate the active compound. The critical parameters of comminution, such as milling and grinding, are mechanical forces, which impart significant amount of physical stress upon the active pharmaceutical ingredient product and may induce degradation. The thermal stress that occurs during comminution is also concern when processing thermo-sensitive or unstable active compounds. Theoretically, with micronization one should be able to achieve large surface areas; however, micronized active pharmaceutical ingredient powders are extremely cohesive due to high energy milling processes that cause significant dislocation of crystal structure on the particle surface. These particles tend to agglomerate during the solid state processing, leading to poor dissolution performance.

In addition, the introduction of a surfactant in a pharmaceutical composition may have a detrimental impact on the stability of the composition. There is also a threshold of feasibility for surfactants. Above certain quantity the surfactants begin to depress solubility. Furthermore, surfactants can disrupt biological membranes - cause fluidization of membrane - and lead to enhanced transcelullar as well as paracellular transport and can on long-term use cause permanent damage to epithelial tissue.

Hence, an improved pharmaceutical composition aiming at the enhanced solubility of the otherwise insoluble or sparingly soluble active pharmaceutical ingredients is needed. By utilizing the embodiments of the present invention, pharmaceutical compositions are provided which enable good solubility in water specifically for sulphonylurea-based active pharmaceutical ingredients, yet without an extensive micronization and/or an addition of surfactants being required.

### Summary of the invention and definitions

First aspect of the present invention is a pharmaceutical composition comprising at least one sulphonylurea-based active pharmaceutical ingredient, wherein the at least one sulphonylurea-based active pharmaceutical ingredient is mixed with a basic agent and the weight ratio of the basic agent to the at least one sulphonylurea-based active pharmaceutical ingredient is from above 1:1 to 50:1.

In an embodiment alternative to, or in combination with, the above first aspect, the pharmaceutical composition comprises the at least one sulphonylurea-based active pharmaceutical ingredient intimately mixed with a basic agent at a weight ratio of the basic agent to the at least one sulphonylurea-based active pharmaceutical ingredient being higher than 1:1, wherein the intimate mixture is obtained by partially or fully dissolving both substances in a solvent and by subsequently at least partially precipitating both substances.

In a further embodiment alternative to, or in combination with, the above first aspect, a basic agent and the sulphonylurea-based active pharmaceutical ingredient are present at a weight ratio of higher than 1:1 and are deposited on hydrophilic particles, wherein the particle size of the particulate hydrophilic diluent is defined by d_{0.9} < 150µm.
Second aspect of the present invention is a tablet comprising glimepiride, **characterized in that** glimepiride is mixed with a basic agent and the weight ratio of the basic agent to glimepiride is from above 1:1 to 50:1, preferably from 1.5:1 to 25:1, more preferably from 2:1 to 15:1, yet more preferably from 3:1 to 10:1

Further aspect of the invention is a process for preparing a pharmaceutical composition comprising at least one sulphonylurea-based active pharmaceutical ingredient, wherein the at least one sulphonylurea-based active pharmaceutical ingredient is admixed with a basic agent, optionally in the presence of a granulation liquid, wherein the weight ratio of the basic agent to the at least one sulphonylurea-based active pharmaceutical ingredient is from above 1:1 to 50:1, preferably from 1.1:1 to 25:1.

Another aspect of the present invention is a process for preparing a pharmaceutical composition comprising at least one sulphonylurea-based active pharmaceutical ingredient, wherein the at least one sulphonylurea-based active pharmaceutical ingredient and a basic agent are partially or fully dissolved in a granulation liquid or a solvent, wherein the weight ratio of the basic agent to the at least one sulphonylurea-based active pharmaceutical ingredient is higher than 1:1, preferably higher than 1.5:1 and more preferably higher than 2:1. In this embodiment, the aforementioned weight ratio is not necessarily limited in the upper mass excess. The upper mass excess limit can be adjusted as desired, inter alia depending on the type of basic agent, its solubility in solvents used and the final solubility in aqueous solutions, the basicity of the final compositions, etc. A suitable upper limit may be 50:1 and preferably is 25:1. The solvent can be used as granulation liquid. After formation of the (partial) solution, the basic agent and the at least one sulphonylurea-based active pharmaceutical ingredient can be at least partly precipitated.

A further aspect of the present invention is a process for preparing pharmaceutical composition comprising at least one sulphonylurea-based active pharmaceutical ingredient, wherein the at least one sulphonylurea-based active pharmaceutical ingredient and a basic agent are partially or fully dissolved in a granulation liquid or a solvent, and subsequently the obtained partial or complete solution is sprayed onto hydrophilic particles selected from the group consisting of modified or unmodified monosaccharides, straight or branched oligosaccharides, and straight or branched polysaccharides, wherein particle size of a particulate hydrophilic diluent is d_{0.9} < 150µm. Preferably hydrophilic diluent is selected from the group consisting of lactose, starch, sucrose, mannitol, sorbitol and cellulose derivatives.

Yet another aspect of the present invention is use of pharmaceutical composition comprising at least one sulphonylurea-based active pharmaceutical ingredient, wherein the at least one sulphonylurea-based active pharmaceutical ingredient is mixed with a basic agent and the weight ratio of the basic agent to the at least one sulphonylurea-based active pharmaceutical ingredient from above 1:1 to 50:1, preferably from 1.1:1 to 25:1, more preferably from 1.5:1 to 20:1, yet more preferably from 2:1 to 15:1, particularly from 3:1 to 10:1, for preparing a medicament.

In preferred embodiments of the above defined aspects of the present invention, the at least one sulphonylurea-based active pharmaceutical ingredient and the basic agent can be beneficially intimately mixed. By "intimately mixed" it is meant herein that active pharmaceutical ingredients, basic agents, optional further excipients or compounds in general are in contact with each other, preferably over a large surface area. This can be achieved by simply mixing said compounds together. Superior result can be achieved when a granulation liquid is used causing that intimately mixed compounds do not get diluted even if a pharmaceutical composition is supplemented with additional excipients. In such case intimately mixed compounds stay intimately mixed even if further excipients are homogenously mixed with intimately mixed compounds, as the additional excipients get mixed only extragranularly. Preferred option is when the granulation liquid causes the compounds intended for intimate mixing to dissolve at least partially. At least partial dissolution in a granulation liquid leads inevitably to providing a contact between compounds over a large surface area during the subsequent simultaneous precipitation of the compounds. Yet more preferred way to achieve intimately mixed compounds it to partially or fully dissolve said compounds in water, at least one organic solvent or mixtures thereof, and at least partly precipitate them. Again, precipitation provides for a large contact surface area between compounds. Precipitation can be achieved for example by drying or spraying technique.

By "sulphonylurea-based active pharmaceutical ingredient" it is meant herewith the insulin secretion enhancer from the sulphonylurea-class of active pharmaceutical ingredients. For example, the sulphonylurea-class of active pharmaceutical ingredients include acetohexamide, chlorpropamide, tolbutamide, tolazamide, glipizide, gliclazide, glibenclamide, gliquidone, glyclopyramide, glimepiride, gliquidone, glixosepid, glimidine, glypinamide, glyhexamide, glibuzole or glybuthiazole. Preferably sulphonylurea-based active pharmaceutical ingredient is selected from the group of glipizide, gliclazide, glibenclamide and glimepiride, more preferably is glimepiride.

By "basic agent" it is meant herein any compound of a pKa value of at least 9 and a solubility in water of at least 10 mg/mL. Preferably the basic agent has a pKa of at least 10, more preferably at least 11. Further, the solubility of the basic agent in water is preferably at least 50 mg/mL, more preferably at least 100 mg/mL. The solubility can be measured at 37°C. Preferably, the basic agent used according to the present invention is selected from a group of organic base, inorganic base or basic amino acid. It is preferably selected from the group consisting of arginine, meglumine, lysine, monoethanolamine, diethanolamine, triethanolamine, propanolamine, dipropanolamine, ammonia, thiamine and sodium salicylate, more preferably is selected from the group of arginine, meglumine, lysine, monoethanolamine, diethanolamine and triethanolamine, yet more preferably is selected from the group of arginine and meglumine. In a specific embodiment, the basic agent in arginine.

By the indication of the weight ratio "above 1:1" or "higher than 1:1" it is meant that the exact ratio 1:1 is exceeded (i.e. exclusive 1:1). The weight ratio 1:1 can be excluded even when normal and typical weighting errors occur.

By "sparingly soluble" it is meant herein the solubility in water of between 10 mg/mL and 33 mg/mL. By "very low solubility" it is meant herein that the solubility in water is lower than "sparingly soluble", it may include substantially insoluble or even insoluble. Solubility as meant herein is measured using USP apparatus 2 with agitation at 75 RPM. The dissolution medium is phosphate buffer maintained at 37 °C.

By "polymer" it is meant herein any polymer suitable for binding, for film coating or for providing a tablet. Examples of such polymers are cellulose derivatives, polyethylene glycols, polyvinylpyrrolidone, polyvinylacetate, polyvinylalcohol, polyesters, polyamides, polyanhydrides, polyorthoesters, polycarbonates, poly(phosphoesters), poly(phosphazenes), poly(iminocarbonates), and the like, mixtures and copolymers thereof. Preferably polymer is selected from the group consisting of cellulose derivatives, polyethylene glycols, polyvinylpyrrolidone, polyvinylacetate, polyvinylalcohol, mixtures and copolymers thereof.

By "hydrophilic diluent" it is meant herein any soluble or not soluble hydrophilic compound, optionally mixed with another at least one pharmaceutically acceptable excipient, present in a form of molecules, particles, powders, granules, beads, tablet cores, or the like, and can be for example any modified or unmodified monosaccharide, straight or branched oligosaccharide, straight or branched polysaccharide or mixtures thereof, wherein the monosaccharide, oligosaccharide or polysaccharide are optionally additionally substituted. The comprehension of the monosaccharide, oligosaccharide or polysaccharide is completely in the purview of the skilled person. For example, the hydrophilic diluent can be lactose, starch, sucrose, mannitol, sorbitol or cellulose derivatives. According to the preferred embodiment of the present invention lactose is used as a hydrophilic diluent. The hydrophilic diluent is preferably present as particles, thereby providing a favorable surface area to deposit the mixture of the at least one sulphonylurea-based active pharmaceutical ingredient and the basic agent in order to provide large surface area, faster wettabilitiy and thereby enhanced solubility in water. A preferred particle size of a particulate hydrophilic diluent is d_{0.9} < 150µm, more preferable d_{0.9} is < 100µm.

### Preferred embodiments of the invention and advantageous combinations thereof

The aspects, advantageous features and preferred embodiments of the present invention summarized in the following items, respectively alone or in combination, further contribute to solving the object of the invention:
1. A pharmaceutical composition comprising at least one sulphonylurea-based active pharmaceutical ingredient, wherein the at least one sulphonylurea-based active pharmaceutical ingredient is mixed with a basic agent and the weight ratio of the basic agent to the at least one sulphonylurea-based active pharmaceutical ingredient is from above 1:1 to 50:1.
2. The pharmaceutical composition according to item 1, wherein the at least one sulphonylurea-based active pharmaceutical ingredient and the basic agent are intimately mixed, preferably by partially or fully dissolving both substances in a solvent and by subsequently at least partially precipitating both substances.
3. A pharmaceutical composition comprising at least one sulphonylurea-based active pharmaceutical ingredient, wherein the at least one sulphonylurea-based active pharmaceutical ingredient is intimately mixed with a basic agent at a weight ratio of the basic agent to the at least one sulphonylurea-based active pharmaceutical ingredient being higher than 1:1, wherein the intimate mixture is obtained by partially or fully dissolving both substances in a solvent and by subsequently at least partially precipitating both substances.
4. A pharmaceutical composition comprising at least one sulphonylurea-based active pharmaceutical ingredient, further comprising a basic agent at a weight ratio of the basic agent to the at least one sulphonylurea-based active pharmaceutical ingredient being higher than 1:1, and wherein the basic agent and the at least one sulphonylurea-based active pharmaceutical ingredient are deposited on hydrophilic diluent particles and/or mixed with hydrophilic diluent particles, wherein the particle size of the particulate hydrophilic diluent is defined by d_{0.9} < 150µm.
5. The pharmaceutical composition according any one of the preceding items, wherein the weight ratio of the basic agent to the at least one sulphonylurea-based active pharmaceutical ingredient is from 1.1:1 to 50:1, preferably from 1.1:1 to 25:1, more preferably from 1.5:1 to 20:1, yet more preferably from 2:1 to 15:1, particularly from 3:1 to 10:1.
6. The pharmaceutical composition according to any one of the preceding items, wherein the basic agent and the at least one sulphonylurea-based active pharmaceutical ingredient are commonly deposited on a hydrophilic diluent and/or commonly homogenously mixed with the hydrophilic diluent.
7. The pharmaceutical composition according to any one of the preceding items, wherein an intimate mixture of the at least one sulphonylurea-based active pharmaceutical ingredient and the basic agent is obtained by partially or fully dissolving both substances in a solvent and subsequently at least partially precipitating both substances by spraying, drying or solvent removal.
8. The pharmaceutical composition according to any one of the preceding items, wherein the hydrophilic diluent is any modified or unmodified monosaccharide, straight or branched oligosaccharide, straight or branched polysaccharide, or mixtures thereof, wherein the monosaccharide, oligosaccharide or polysaccharide are optionally additionally substituted; preferably the hydrophilic diluent is selected from the group consisting of lactose, starch, sucrose, mannitol, sorbitol and cellulose derivatives.
9. The pharmaceutical composition according to any one of the preceding items, wherein the composition further comprises at least one polymer.
10. The pharmaceutical composition according to item 9, wherein the polymer is selected from the group consisting of cellulose derivatives, polyethylene glycols, polyvinylpyrrolidone, polyvinylacetate, polyvinylalcohol, polyesters, polyamides, polyanhydrides, polyorthoesters, polycarbonates, poly(phosphoesters), poly(phosphazenes), poly(iminocarbonates), mixtures and copolymers thereof, preferably polymer is selected from the group consisting of cellulose derivatives, polyethylene glycols, polyvinylpyrrolidone, polyvinylacetate, polyvinylalcohol, mixtures and copolymers thereof.
11. The pharmaceutical composition according to any one of the preceding items, wherein the composition is formulated in a dosage form.
12. The pharmaceutical composition according to item 11, wherein the dosage form is a powder, granulate, tablet, at least one layer of a double or multilayer tablet, film coating, mantle coating, pellet, capsule, preferably tablet, at least one layer of a double or multilayer tablet or film coating, more preferably tablet, wherein each of the obtained formulations is optionally film coated or mantle coated.
13. The pharmaceutical composition according to item 12, defined by a core comprising said at least one sulphonylurea-based active pharmaceutical ingredient and a film coat or a mantle coat, wherein the film coat or mantle coat comprises at least one active pharmaceutical ingredient other than the sulphonylurea-based active pharmaceutical ingredient of the core, or defined by a core and a film coat or a mantle coat, wherein the film coat or the mantle coat comprises said at least one sulphonylurea-based active pharmaceutical ingredient and the core comprises at least one active pharmaceutical ingredient other than the sulphonylurea-based active pharmaceutical ingredient of the film coat or the mantle coat, or defined by two or more layers, wherein the at least one first layer comprises said at least one sulphonylurea-based active pharmaceutical ingredient and the same or other at least one layer comprises at least one active pharmaceutical ingredient other than the sulphonylurea-based active pharmaceutical ingredient of the said first layer.
14. The pharmaceutical composition according to any one of the preceding items, wherein the composition further comprises at least one active pharmaceutical ingredient selected from the group consisting of thiazolidinone class compounds, biguanide class compounds, statin class compounds, fibrate class compounds, acarbose, miglitol, voglibose, orlistat, sibutramine, rimonabant, exenatide and pramlintide; preferably selected from biguanide class compounds and thiazolidinone class compounds, more preferably thiazolidinone class compounds, particularly further active pharmaceutical ingredient is pioglitazone or rosiglitazone.
15. The pharmaceutical composition according to any one of items 1 to 13, wherein the composition further comprises at least one active pharmaceutical ingredient of thiazolidinone class compounds or biguanide class compounds, preferably thiazolidinone class compounds.
16. The pharmaceutical composition according to any one of items 1 to 13, wherein the composition further comprises at least one active pharmaceutical ingredient of thiazolidinone class compounds.
17. The pharmaceutical composition according to any one of items 1 to 13, wherein the composition further comprises at least one active pharmaceutical ingredient of statin class compounds or fibrate class compounds, preferably statin class compounds.
18. The pharmaceutical composition according to any one of items 1 to 13, wherein the composition further comprises at least one active pharmaceutical ingredient selected from acarbose, miglitol, voglibose, orlistat, sibutramine, rimonabant, exenatide and pramlintide, preferably selected from acarbose, miglitol, voglibose, orlistat, more preferably from acarbose.
19. The pharmaceutical composition according to any one of the preceding items, wherein the at least one sulphonylurea-based active pharmaceutical ingredient is selected from the group of acetohexamide, chlorpropamide, tolbutamide, tolazamide, glipizide, gliclazide, glibenclamide, gliquidone, glyclopyramide, glimepiride, gliquidone, glixosepid, glimidine, glypinamide, glyhexamide, glibuzole and glybuthiazole, preferably from the group of glipizide, gliclazide, glibenclamide and glimepiride, most preferably is glimepiride.
20. The pharmaceutical composition according to any one of the preceding items, wherein the basic agent is selected from the group consisting of arginine, meglumine, lysine, monoethanolamine, diethanolamine, triethanolamine, propanolamine, dipropanolamine, ammonia, thiamine and sodium salicylate, preferably is selected from the group consisting of arginine, meglumine, lysine, monoethanolamine, diethanolamine and triethanolamine, yet more preferably is selected from the group of arginine and meglumine.
21. A tablet comprising glimepiride, **characterized in that** glimepiride is mixed with a basic agent and the weight ratio of the basic agent to glimepiride is from above 1:1 to 50:1, preferably from 1.1:1 to 25:1, more preferably 1.5:1 to 20:1, yet more preferably from 2:1 to 15:1, particularly from 3:1 to 10:1.
22. The tablet according to item 21, wherein glimepiride and the basic agent are intimately mixed, preferably by partially or fully dissolving both substances in a solvent and by subsequently at least partially precipitating both substances.
23. The tablet according to item 21 or 22, wherein the basic agent is selected from the group consisting of arginine, meglumine, lysine, monoethanolamine, diethanolamine, triethanolamine, propanolamine, dipropanolamine, ammonia, thiamine and sodium salicylate, more preferably is selected from the group of arginine, meglumine, lysine, monoethanolamine, diethanolamine and triethanolamine, yet more preferably is selected from the group of arginine and meglumine.
24. The tablet according to any one of items 21 to 23, further comprising a hydrophilic diluent.
25. The tablet according to item 24, wherein the hydrophilic diluent is any modified or unmodified monosaccharide, straight or branched oligosaccharide, straight or branched polysaccharide, or mixtures thereof, wherein the monosaccharide, oligosaccharide or polysaccharide are optionally additionally substituted.
26. The tablet according to any one of items 21 to 25, further comprising at least one further active pharmaceutical ingredient selected from the group consisting of thiazolidinone class compounds, preferably pioglitazone or rosiglitazone, more preferably pioglitazone.
27. The pharmaceutical composition according to any one of items 4 to 20 or the tablet according to any one of items 24 to 26, wherein the hydrophilic diluent is lactose.
28. The tablet according to any one of items 21 to 27, wherein the tablet further comprise at least one polymer, wherein the polymer is selected from the group consisting of cellulose derivatives, polyethylene glycols, polyvinylpyrrolidone, polyvinylacetate, polyvinylalcohol, polyesters, polyamides, polyanhydrides, polyorthoesters, polycarbonates, poly(phosphoesters), poly(phosphazenes), poly(iminocarbonates), mixtures and copolymers thereof, preferably polymer is selected from the group consisting of cellulose derivatives, polyethylene glycols, polyvinylpyrrolidone, polyvinylacetate, polyvinylalcohol, mixtures and copolymers thereof.
29. The pharmaceutical composition according to any one of items 9 to 20 or the tablet according to item 28, wherein the polymer is hydroxypropylcellulose.
30. A process for preparing a pharmaceutical composition comprising at least one sulphonylurea-based active pharmaceutical ingredient, wherein the at least one sulphonylurea-based active pharmaceutical ingredient is admixed with a basic agent, optionally in the presence of a granulation liquid or a solvent, wherein the weight ratio of the basic agent to the at least one sulphonylurea-based active pharmaceutical ingredient is from above 1:1 to 50:1.
31. The process according to item 30, wherein the weight ratio of the basic agent and the at least one sulphonylurea-based active pharmaceutical ingredient is from 1.5:1 to 25:1, preferably from 2:1 to 15:1, more preferably from 3:1 to 10:1.
32. The process according to any one of items 30 and 31, wherein the at least one sulphonylurea-based active pharmaceutical ingredient and the basic agent are intimately mixed by partially or fully dissolving both substances in a solvent and by subsequently at least partially precipitating both substances.
33. The process according to item 32, wherein said at least partially precipitating both substances is carried out by spraying, drying or solvent removal.
34. A process for preparing a pharmaceutical composition comprising at least one sulphonylurea-based active pharmaceutical ingredient, wherein the at least one sulphonylurea-based active pharmaceutical ingredient and a basic agent are partially or fully dissolved in a granulation liquid or a solvent, wherein the weight ratio of the basic agent and the at least one sulphonylurea-based active pharmaceutical ingredient is higher than 1:1, and subsequently both substances are at least partly precipitated.
35. The process according to any one of items 30 to 34, wherein the at least one sulphonylurea-based active pharmaceutical ingredient and a basic agent are deposited on hydrophilic diluent and/or homogenously mixed with the hydrophilic diluent.
36. The process according to any one of items 30 to 35, wherein the at least one sulphonylurea-based active pharmaceutical ingredient and a basic agent are coated on pharmaceutical core.
37. A process for obtaining a pharmaceutical composition comprising at least one sulphonylurea-based active pharmaceutical ingredient, wherein the at least one sulphonylurea-based active pharmaceutical ingredient and a basic agent are partially or fully dissolved in a granulation liquid or a solvent, and subsequently the obtained partial or complete solution is sprayed onto hydrophilic particles and/or intimately mixed with hydrophilic particles, the hydrophilic particles being selected from the group consisting of modified or unmodified monosaccharides, straight or branched oligosaccharides, and straight or branched polysaccharides, wherein the particle size of the particulate hydrophilic diluent is defined by d_{0.9} < 150µm.
38. The process according to item 37, wherein the hydrophilic diluent is any modified or unmodified monosaccharide, straight or branched oligosaccharide, straight or branched polysaccharide, or mixtures thereof, wherein the monosaccharide, oligosaccharide or polysaccharide are optionally additionally substituted; preferably the hydrophilic diluent is selected from the group consisting of lactose, starch, sucrose, mannitol, sorbitol and cellulose derivatives.
39. The process according to any one of items 34 to 38, wherein the weight ratio of the basic agent to the at least one sulphonylurea-based active pharmaceutical ingredient is from above 1 to 50:1, preferably from 1.5:1 to 25:1, more preferably from 2:1 to 15:1, yet more preferably from 3:1 to 10:1.
40. The process according to any one of items 30 to 39, wherein the granulation liquid or solvent is selected from water, at least one organic solvent or mixtures thereof.
41. The process according to any one of items 30 to 40, wherein the organic solvent is alkyl halide or straight or branched alcohol of less than 8 carbon atoms, preferably straight or branched alcohol of less than 5 carbon atoms.
42. The process according to any one of items 30 to 40, wherein the organic solvent is selected from the group of dichloromethane, methanol, ethanol, propanol, isopropanol, butanol and terc-butanol, preferably ethanol and isopropanol, more preferably ethanol.
43. The process according to any one of items 30 to 40, wherein the granulation liquid is a mixture of water and straight or branched alcohol of less than 5 carbon atoms, preferably of water and ethanol or isopropanol, more preferably water and ethanol.
44. The process according to any one of items 30 to 43, wherein a polymer is further added to the composition.
45. The process according to any one of items 30 to 44, wherein the process further comprises adding pharmaceutical acceptable excipients and/or at least one other active pharmaceutical ingredient selected from the group consisting of thiazolidinone class compounds, biguanide class compounds, statin class compounds, fibrate class compounds, acarbose, miglitol, voglibose, orlistat, sibutramine, rimonabant, exenatide and pramlintide, preferably selected from biguanide class compounds and thiazolidinone class compounds, more preferably thiazolidinone class.
46. The process according to item 45, wherein the at least one other active pharmaceutical ingredient is selected from the group consisting of meglumine, pioglitazone and rosiglitazone, preferably pioglitazone.
47. The process according to any one of items 30 to 46, wherein further polymer is added to a solvent comprising water, at least one organic solvent or mixtures thereof.
48. The process according to any one of items 30 to 46, wherein the polymer is selected from the group consisting of cellulose derivatives, polyethylene glycols, polyvinylpyrrolidone, polyvinylacetate, polyvinylalcohol, polyesters, polyamides, polyanhydrides, polyorthoesters, polycarbonates, poly(phosphoesters), poly(phosphazenes), poly(iminocarbonates), mixtures and copolymers thereof, preferably polymer is selected from the group consisting of cellulose derivatives, polyethylene glycols, polyvinylpyrrolidone, polyvinylacetate, polyvinylalcohol, mixtures and copolymers thereof.
49. The pharmaceutical composition according to any one of items 1 to 20, 27 and 29, the tablet according to any one of items 21 to 26, 28 and 29, or the process according to any one of items 30 to 48, wherein the basic agent has a pKa over 9 and a solubility in water of at least 10 mg/mL.
50. The pharmaceutical composition according to any one of items 1 to 20, 27, 29 and 49, the tablet according to any one of items 21 to 26, 28, 29 and 49, or the process according to any one of items 30 to 49, wherein the basic agent is selected from the group of organic base, inorganic base and basic amino acid, is preferably selected from the group of arginine, meglumine, lysine, monoethanolamine, diethanolamine, triethanolamine, propanolamine, dipropanolamine, ammonia, thiamine and sodium salicylate, more preferably is selected from the group of arginine, meglumine, lysine, monoethanolamine, diethanolamine and triethanolamine, yet more preferably is selected from the group of arginine and meglumine.
51. The pharmaceutical composition according to any one of items 1 to 20, 27, 29, 49 and 50, the tablet according to any one of items 21 to 26, 28, 29, 49 and 50, or the process according to any one of items 30 to 50, without a further size reduction or micronization of the starting active pharmaceutical ingredient being required, and/or without an addition of surfactants being required.
52. The process according to any one of items 30 to 48, wherein glimepiride and arginine are dissolved in a weight ratio from above 1:1 to 50:1, preferably from 1.5:1 to 25:1, more preferably from 2:1 to 15:1, yet more preferably from 3:1 to 10:1, in a mixture of ethanol and water sprayed onto lactose, and optionally further admixed with excipients or at least one other active pharmaceutical ingredient.
53. Use of pharmaceutical composition comprising at least one sulphonylurea-based active pharmaceutical ingredient, wherein the at least one sulphonylurea-based active pharmaceutical ingredient is mixed with a basic agent and the weight ratio of the basic agent to the at least one sulphonylurea-based active pharmaceutical ingredient is from above 1:1 to 50:1, preferably from 1.5:1 to 25:1, more preferably from 2:1 to 15:1, yet more preferably from 3:1 to 10:1, for preparing a medicament.
54. The use according to item 53, wherein the at least one sulphonylurea-based active pharmaceutical ingredient is selected from the group consisting of acetohexamide, chlorpropamide, tolbutamide, tolazamide,glipizide, gliclazide, glibenclamide, gliquidone, glyclopyramide, glimepiride, gliquidone, glixosepid, glimidine, glypinamide, glyhexamide, glibuzole and glybuthiazole, preferably from the group consisting of glipizide, gliclazide, glibenclamide and glimepiride, most preferably is glimepriride.
55. The use according to any one of items 53 and 54, wherein the basic agent has a pKa over 9 and a solubility in water of at least 10 mg/mL.
56. The use according to any one of items 53 to 55, wherein the basic agent is selected from the group consisting of organic base, inorganic base and basic amino acid, and is preferably selected from the group consisting of arginine, meglumine, lysine, monoethanolamine, diethanolamine, triethanolamine, propanolamine, dipropanolamine, ammonia, thiamine and sodium salicylate, more preferably is selected from the group consisting of arginine, meglumine, lysine, monoethanolamine, diethanolamine and triethanolamine, yet more preferably is selected from the group of arginine and meglumine.
57. The use according to any one of items 53 to 56, wherein the basic agent and the at least one sulphonylurea-based active pharmaceutical ingredient are intimately mixed by partially or fully dissolving both substances in a granulation liquid or a solvent, followed by deposition on a hydrophilic diluent and/or formation of homogenous mixture with a hydrophilic diluent.
58. The use according to any one of items 53 to 57, wherein the composition further comprises at least one polymer.

### Brief description of the drawings

Fig. 1: Dissolution study in a phosphate buffer with pH 6.8 of different compositions of example 1
Fig. 2: Dissolution study of different compositions obtained as disclosed in examples 1 to 4.
Fig. 3: Dissolution study of different compositions obtained as disclosed in examples 3 and 5 to 8.

### Detailed description of the invention

Sulphonylurea-based active pharmaceutical ingredients have very low and pH-dependent solubility. For example, in acidic medium glimepiride exhibits the solubility of less than 0.001 mg/mL at 37 °C. In media with pH around 7 the solubility of glimepiride is 0.001 mg/mL, of gliclazide is 0.05 mg/mL, of glipizide is 0.037 mg/mL and of glibenclamide is 0.06 mg/mL. At a pH 8, the solubility of glimepiride only slightly increases to 0,03 mg/mL.

Surprisingly it was discovered that the solubility of the sulphonylurea-based active pharmaceutical ingredients can be significantly increased by simply mixing the sulphonylurea-based active pharmaceutical ingredients with a sufficient amount of a basic agent. Sufficient amount of a basic agent to achieve enhanced dissolution is reached when a weight ratio of the basic agent to the sulphonylurea-based active pharmaceutical ingredients is in excess of 1:1 and in particular when selecting a range of from above 1:1 to 50:1 such as from 1.1:1 to 50:1, preferably from 1.5:1 to 25:1, more preferably from 2:1 to 15:1, yet more preferably from 3:1 to 10:1. During dissolution, this ratio enables the basic agent to provide for a particles of the sulphonylurea-based active pharmaceutical ingredients an alkaline microenvironment, causing the sulphonylurea-based active pharmaceutical ingredients to dissolve extremely fast and to the larger extent in aqueous solutions.

According to an alternative solution to the solubility problem, surprisingly a controlled intimate mixing of the basic agent with the at least one sulphonylurea-based active pharmaceutical ingredient at a weight ratio higher than 1:1 drastically enhances the solubility. According to this embodiment partially or fully dissolving both substances in a solvent and subsequently at least partially precipitating both substances is a particularly efficient and beneficial way to obtain the intimate mixture.
In connection with still another solution to the solubility problem of the sulphonylurea-based active pharmaceutical ingredient, it has been surprisingly discovered that when the at least one sulphonylurea-based active pharmaceutical ingredient and the basic agent are, in an intimately mixed state, deposited on hydrophilic diluent and/or intimately mixed with hydrophilic diluent, the solubility can likewise be drastically enhanced; even more so when hydrophilic diluent is in a particulate form and the particle size of the particulate hydrophilic diluent is defined by d_{0.9} < 150µm. The hydrophilic diluent particles are preferably selected from the group consisting of modified or unmodified monosaccharides, straight or branched oligosaccharides, and straight or branched polysaccharides.

Unexpectedly, the improvements of the present invention can be achieved without the need to observe a particular particle size of the sulphonylurea-based ingredients. Rather, a given sulphonylurea-based ingredient can be used as starting substance independent from size selection, and without a need for further size reduction or further micronization. This provides for a bypass around the need for reducing the particle size, relieving the process of increased physical or thermal stress as this are normally possible causes of deterioration. The improvement considerably adds to the stability of the pharmaceutical composition. In addition, the introduction of high energy into the composition, which is characteristic for milling is with utilizing the present invention completely dispensable. Thus, there is no danger of agglomeration during the solid state processing, which would lead to poor dissolution performance. Further, it is an advantage to enable the use of any sulphonylurea-based ingredient material, no matter what size it has, or without a need to pay attention to its size range.

In addition, as the particle size is no longer of importance, the pharmaceutical composition of the present invention is suitable for being formulated in any dosage form, and yet it will provide fast dissolution rate of the sulphonylurea-based active pharmaceutical ingredient, which was not the case in the so far existing dosage forms; for example with tablets micronized glimepiride was essential for satisfactory dissolution rate.

Moreover, the present invention makes all pitfalls arising from the addition of the surfactant superfluous, since the employment of the surfactant in the pharmaceutical composition is only optional and not necessary for achieving high dissolution rates.

In addition, raising of the weight ratio of the basic agent relative to the sulphonylurea-based active pharmaceutical ingredients to a mass excess of higher than 1:1, for example at least 1.1:1, preferably to a range of 1.1:1 to 50:1, more preferably 1.5:1 to 20:1, to 2:1, yet more preferably to 3:1 surprisingly proved to be advantageous over prior art teaching. In particularly preferred embodiments, it enables the pharmaceutical compositions to comprise sulphonylurea-based active pharmaceutical ingredient and another at least one active pharmaceutical ingredient homogenously mixed. Prior art discloses the pharmaceutical dosage forms comprising sulphonylurea-based active pharmaceutical ingredient and another at least one active pharmaceutical ingredient, wherein different active ingredients are distributed to separate compartments of the dosage forms due to instability issues. Sulphonylurea-based active pharmaceutical ingredients have amide and sulfone-amide groups, which hydrolyze in the presence of acidic compound and pose stability problems. However, mixing of the basic agent with the sulphonylurea-based active pharmaceutical ingredients in the weight ratio from above 1:1, such as 1.1:1, preferably from 1.5:1 or more, enables association of sulphonylurea-based active pharmaceutical ingredients together with another at least one acidic active pharmaceutical ingredients in the same pharmaceutical composition or further in the same compartment of the dosage form. It is understood that also acidic salts of another at least one active pharmaceutical ingredients can be associated with the intimately mixed sulphonylurea-based active pharmaceutical ingredient and the basic agent in the same pharmaceutical composition or the dosage form compartment if the weight ratio criteria is met.

Yet, it is alternatively possible to separate different active pharmaceutical ingredients and to include them into different compartments of a pharmaceutical composition. For example, the pharmaceutical composition may comprise a core and a film coat or a mantle coat, or may be defined by multi-layers, and said at least one sulphonylurea-based active pharmaceutical ingredient is incorporated either into the core, or into the film coat or a mantle coat, or in at least one layer of the multi-layers, whereas the other active pharmaceutical ingredient is incorporated into the other compartment(s).

However, increasing the ratio of the basic agent and the at least one sulphonylurea-based active pharmaceutical ingredients to more than 25:1, and more pronounced in case of more than 50:1 leads to pharmaceutical composition having improper handling characteristics without substantial gain in solubility enhancement. Increasing the amount of basic compound above threshold of 25:1 and even more so above 50:1 may make the pharmaceutical composition too basic and therefore corrosive, which makes is unsuitable for normal handling and administration. In certain embodiments with further solubility enhancement concepts, the weight ratio may nevertheless be increased to above 50:1, for example up to the solubility limit of the alkaline agent in water. Problem of alkalinity and corrosiveness may be counteracted, for example by the provision of a protective film coat, and acid resistant coating and the like.

The pharmaceutical composition can be obtained by simply mixing sulphonylurea-based active pharmaceutical ingredient and the basic agent in the aforementioned weight ratio. Preferably, the mixing is performed in the presence of the granulation liquid. The granulation liquid can be water, at least one organic solvent or mixtures thereof. The organic solvent can be for example alkyl halide or straight or branched alcohol of less than 8 carbon atoms or mixtures thereof, preferably straight or branched alcohol of less than 5 carbon atoms. Particularly preferred, the organic solvent is for example dichloromethane, methanol, ethanol, propanol, isopropanol, butanol, terc-butanol. Specifically suitable granulation liquids are mixtures of water and alcohol of less than 5 carbon atoms, preferably are mixtures of water and ethanol or isopropanol. The use of granulation liquid in this specific settings contributes to more compact mixture of the at least one sulphonylurea-based active pharmaceutical ingredient and the basic agent, leading to the result that the compounds remain intimately mixed to higher extent even after optionally adding further excipients or another at least one pharmaceutical active ingredient while further mixing the pharmaceutical composition or formulating the composition to dosage form. The process of mixing can be simple mixing, blending or co-milling or the like. The process can be performed in any apparatus customary used for mixing. For example it can be done in a high shear mixer.

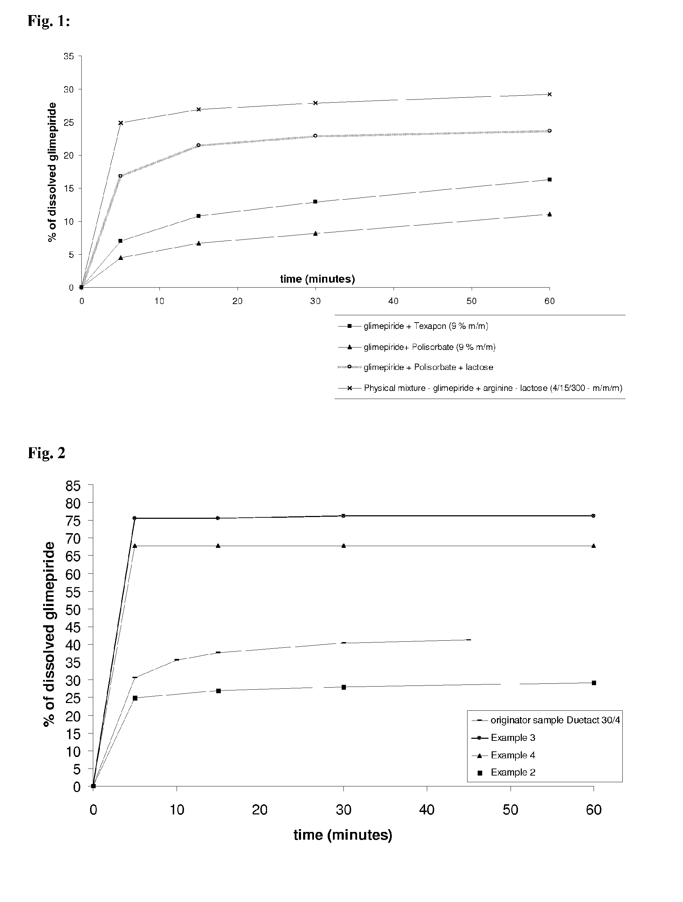

As an alternative to, optionally in combination with, the above process, superior result can be achieved when the at least one sulphonylurea-based active pharmaceutical ingredient and the basic agent are at least partially or fully dissolved in a granulation liquid or a solvent, wherein the weight ratio of the basic agent and the at least one sulphonylurea-based active pharmaceutical ingredient is as described above, and then at least partly precipitated. The granulation liquid or solvent chosen for at least partly or fully dissolving compound can be as above water, at least one organic solvent or mixtures thereof. Particularly useful are the organic solvents like for example dichloromethane, methanol, ethanol, propanol, isopropanol, butanol, terc-butanol. Specifically suitable granulation liquids or solvents for at least partly dissolving the compounds are mixtures of water and alcohol of less than 5 carbon atoms, preferably are mixtures of water and ethanol or isopropanol, yet more preferably is the mixture of water and ethanol. According to the preferred process, the at least one sulphonylurea-based active pharmaceutical ingredient and the basic agent, respectively at least partially or fully dissolved, subsequently are allowed to at least partially precipitate. The precipitation can be achieved by mixing technique, spraying techniques, drying, solvent removal technique, or the like. In one embodiment, the at least partly or fully dissolved compounds are sprayed to form granulate. Common precipitation of sulphonylurea-based active pharmaceutical ingredient and the basic agent from the same granulation liquid or solvent allows compounds to be intimately mixed by getting mixed over a large contact area. This embodiment provides for advantageous effect of improved solubility and dissolution rate, as demonstrated by representatively exemplifying samples shown in Fig. 2. Using the granulation liquid also adds to better characteristics of the obtained pharmaceutical composition in terms of improved flowability and compressibility of the pharmaceutical composition, which in turn eases the formulation of the pharmaceutical composition in a dosage form.

Both processes, either the mixing of compounds, optionally in the presence of a granulation liquid, or at least partly or fully dissolving the compounds in the granulation liquid or solvent and at least partly precipitating the compounds, allow for improving the dissolution of the sulphonylurea-based active pharmaceutical ingredient by depositing the compounds on hydrophilic diluent and/or homogenously mixing compounds with the hydrophilic diluent. Hydrophilic diluent further promotes the solubilizing properties of the pharmaceutical composition and increases the contact surface area of the sulphonylurea-based active pharmaceutical ingredient and the basic agent. Both features advance the dissolution rate of the sulphonylurea-based active pharmaceutical ingredient.

As a further alternative to, optionally in combination with, the above process, superior result can be achieved when the particle size of the particulate hydrophilic diluent is defined by d_{0.9} < 150µm, the solubility can likewise be drastically enhanced. Preferred particle size is d_{0.9} < 100µm. The hydrophilic diluent is preferably selected from the group consisting of modified or unmodified monosaccharides, straight or branched oligosaccharides, and straight or branched polysaccharides, or mixtures thereof, respectively in particulate form. Preferably hydrophilic diluent is selected from the group consisting of lactose, starch, sucrose, mannitol, sorbitol and cellulose derivatives. Lactose is particularly preferred.

In another embodiment of the present invention at least one polymer can be added to the mixture of the at least one sulphonylurea-based active pharmaceutical ingredient and the basic agent. The polymer is preferably added to the liquid when the compounds are at least or fully dissolved in the granulation liquid or solvent. Polymer provides for more robust process of granulation or spraying. Preferably, hydroxypropylcellulose or hydroxypropylmethylcellulose are used as the polymer. The polymer can also promote depositing of the compounds on the hydrophilic diluent and/or homogenously mixing compounds with the hydrophilic diluent. It increases yield of depositing when for example spraying the at least partly dissolved at least one sulphonylurea-based active pharmaceutical ingredient and the basic agent on the hydrophilic diluent and reduces loses of compounds during the processing, especially when lactose is used as the hydrophilic diluent. In addition, the attributes of adding the polymer in the pharmaceutical composition lead to improved solubility of the sulphonylurea-based active pharmaceutical ingredient.

According to the present embodiment, the pharmaceutical compositions disclosed herein, both as the product as such or during its production process, can be supplemented with additional excipients. Suitable excipients are any conventionally used pharmaceutically acceptable excipients. For example, the excipients are filler (lactose, mannitol, cellulose derivatives, sucrose, etc.), disintegrator (crosscarmellose sodium, calcium carbonate, carboxymethylcellulose calcium etc.), binder (polyvinylpyrrolidone, etc.), a lubricant (magnesium stearate, stearic acid, silicium dioxide, etc.), fragrant, colorant (titanium dioxide, etc.), a sweetener (saccharine, etc.) or coating polymers (hydroxypropylmethylcellulose, etc.), vehicles (water, organic solvents, etc.). Further, the pharmaceutical composition according to the present embodiment can be formulated in a dosage form. Dosage form can be powder, granulate, tablet, at least one layer of a double or multilayer tablet, film coating, mantle coating, pellet, capsule, semi-solid formulation, suspension, solution or the like, wherein the obtained formulations is optionally film coated or mantle coated. Preferably it is tablet, at least one layer of a double or multilayer tablet or film coating, more preferably tablet. The preparation of dosage form will be immediately evident to the person skilled in the art. For example, the pharmaceutical composition according to the present invention is filled in the gelatine or hypromellose capsules. The composition may be directly compressed or first granulated and then compressed on the tablet press to obtain tablets. Double- or multilayer-mode can be used on the tablet press to prepare double- or multi-layered tablets. Also, tablet press can be used to obtain mantle coating. Film coating, on the other hand, can be prepared by dissolving or dispersing the pharmaceutical composition in a suitable vehicle for film coating and depositing the vehicle on the cores. Various spheronization or extrusion techniques can be employed to prepare pellets. Optionally, each dosage form is further mantle coated or film coated. Film coating can be prepared by dissolving or dispersing suitable polymer for coating and if necessary plasticizers, stabilizers (antioxidants, acidic or basic agents, etc.) or the like in a vehicle and depositing the vehicle on the dosage form. The process can be done for example by spraying technique or dip coating. Example of the apparatus used is a coating pan. Mantle coating can be achieved by compacting the pharmaceutical formulation around the dosage form. In a specific embodiment, film coat or mantle coat comprises at least one active pharmaceutical ingredient.

The pharmaceutical composition, or the film coat or the mantle coat can in addition to the at least one sulphonylurea-based active pharmaceutical ingredient further comprise another at least one active pharmaceutical ingredient. The added other at least one active pharmaceutical ingredient can be selected from thiazolidinone class, biguanide class, statin class, fibrate class, acarbose, miglitol, voglibose, orlistat, sibutramine, rimonabant, exenatide and pramlintide, is preferably selected from biguanide class and thiazolidinone class, more preferably is from thiazolidinone class. Active pharmaceutical ingredient from a thiazolidinone class are for example pioglitazone, rivoglitazone, rosiglitazone, troglitazone. Preferably thiazolidinone class active pharmaceutical ingredient is pioglitazone or rosiglitazone, more preferably pioglitazone. Active pharmaceutical ingredient from the biguanide class are for example phenformin, buformin or metformin. Preferably biguanide class active pharmaceutical ingredient is metformin. Active pharmaceutical ingredient from the statin class are for example atorvastatin, cerivastatin, fluvastatin, lovastatin, mevastatin, pitavastatin, pravastatin, rosuvastatin or simvastatin. Preferably statin class active pharmaceutical ingredient is atorvastatin, pravastatin, rosuvastatin or simvastatin, more preferably rosuvastatin. Active pharmaceutical ingredient from the fibrate class are for example bezafibrate, ciprofibrate, clofibrate, gemfibrozil or fenofibrate. Preferably fibrate class active pharmaceutical ingredient is gemfibrozil or fenofibrate, more preferably fenofibrate.

A specific embodiment of the present invention is the pharmaceutical composition comprising at least one sulphonylurea-based active pharmaceutical ingredient, wherein the at least one sulphonylurea-based active pharmaceutical ingredient is intimately mixed with a basic agent and the weight ratio of the basic agent and the at least one sulphonylurea-based active pharmaceutical ingredient is from 1.1:1 to 50:1, preferably from 1.5:1 to 20:1, more preferably from 2:1 to 15:1, yet more preferably from 3:1 to 10:1, and a hydrophilic diluent, and optionally a polymer. In another specific embodiment the at least one sulphonylurea-based active pharmaceutical ingredient is glimepiride.

Even more preferred embodiment is a tablet comprising glimepiride, wherein glimepiride is intimately mixed with a basic agent and the weight ratio of the basic agent and glimepiride is from 1.1:1 to 50:1, preferably from 1.5:1 to 20:1, more preferably from 2:1 to 15:1, yet more preferably from 3:1 to 10:1. The tablet can further comprise a hydrophilic diluent. The hydrophilic diluent is preferably lactose. The tablet can further comprise at least one polymer. The polymer is selected from the group consisting of cellulose derivatives, polyethylene glycols, polyvinylpyrrolidone, polyvinylacetate, polyvinylalcohol, polyesters, polyamides, polyanhydrides, polyorthoesters, polycarbonates, poly(phosphoesters), poly(phosphazenes), poly(iminocarbonates), mixtures and copolymers thereof. Preferably polymer is selected from the group consisting of cellulose derivatives, polyethylene glycols, polyvinylpyrrolidone, polyvinylacetate, polyvinylalcohol, mixtures and copolymers thereof. In addition, the tablet can further comprise at least one active pharmaceutical ingredient of thiazolidinone class, preferably pioglitazone or rosiglitazone, more preferably pioglitazone. Both active agents can be beneficially combined in the same dosage form, i.e. without a need for separation of the active ingredients.

The aforementioned pharmaceutical composition or a dosage form of any embodiment, alone or in any combination is suitable for preparing a medicament. The process steps necessary for preparing the medicament will be immediately evident to the person skilled in the art when the pharmaceutical formulation, dosage form and recipient are taken into consideration. In general, steps like determining the dose, administration route, choosing of the suitable pharmaceutical composition and/or dosage form and packaging are undertaken when preparing the medicament.

The following examples are merely illustrative of the present invention and they should not be considered as limiting the scope of the invention in any way, as these examples, modifications and other equivalents thereof will become apparent to those versed in the art in the light of the present disclosure, and the accompanying claims.

### Example 1

Various pharmaceutical compositions comprising sulphonylurea-based active pharmaceutical ingredient (glimepiride), accepted surfactant (Texapon (sodium lauryl sulfate), Polisorbate or basic agent (arginine), optionally further comprising hydrophilic diluent (lactose), were prepared and tested for increased solubility of glimepiride. Results are presented in the Fig. 1.

| **Sample** | **Process** | **Pharmaceutical composition** |
|---|---|---|
| **1** | Physical mixture | glimepiride and Texapon (91/9 % m/m) |
| **2** | Physical mixture | glimepiride and Polisorbate (91/9 % m/m) |
| **3** | Granulate | glimepiride, Polisorbate and lactose (4/1/95 % m/m) |
| **4** | Physical mixture | glimepiride, arginine and lactose (1.3/4.7/94.0 % m/m) |

The dissolution study evidently shows that already physically mixed arginine with glimepiride promotes the dissolution rate of the glimepiride to a greater extent than the various surfactants do.

Conditions of testing: dissolution apparatus, paddle (75 rpm), 37 °C, phosphate buffer pH=6,8 (900 mL)

### HPLC conditions:

Waters system equipped with a C18, 3,5 µm, 150 x 4,6 mm column (XBridge) which was maintained in a column oven at 30 °C. The mobile phase A consisted of a mixture of a phosphate buffer of pH 2,5 and acetonitrile in ratio 72 : 28 (V/V). The mobile phase B consisted of a mixture of a phosphate buffer of pH 2,5 and acetonitrile in ratio 30 : 70 (V/V). The flow rate was 1.5 ml/min and the detection wavelength 230 nm. Solvent mixture consisted of water and acetonitrile in the ratio 200 : 800 (V/V). Stock solution was prepared in concentration of 0,02 mg/mL

### Gradient

| time (min) | % A |
|---|---|
| 0 | 100 |
| 2 | 0 |
| 5 | 0 |
| 5,5 | 100 |

### Examples 2 to 4

Three pharmaceutical compositions comprising sulphonylurea-based active pharmaceutical ingredient (glimepiride), basic agent (arginine), and hydrophilic diluent (lactose) were prepared either by simply mixing or by using the granulation liquid as indicated in the following table. In example 2, the glimepiride, arginine and lactose were mechanically mixed. In examples 3 and 4, the glimepiride and arginine were dissolved in the granulation liquid and deposited on the lactose particles.

| **Composition\ Process** | **Example 2** | **Example 3** | **Example 4** |
|---|---|---|---|
| | Physical mixture | granulation | granulation |
| **glimepiride** | 4 mg | 4 mg | 4 mg |
| **Arginine** | 15 mg | 10 mg | 15 mg |
| **Lactose** | 300 mg | 300 mg | 300 mg |
| **Water** | - | 500 mg | 500 mg |
| **Ethanol** | - | 500 mg | 500 mg |

Tested reference product Duetact 30/4 consisted of the following excipients: polisorbate 80, crosscarmellose sodium, lactose monohydrate, hydroxypropy cellulose and microcrystalline cellulose. The dissolution study of the described pharmaceutical compositions and the reference product was performed under the same testing conditions as in example 1. Results are presented in the Fig. 2.

The dissolution study evidently shows the superior dissolution rate of the glimepiride when used with basic agent (arginine).

### Examples 5 to 8

Four pharmaceutical compositions comprising sulphonylurea-based active pharmaceutical ingredient (glimepiride), basic agent (arginine), with and without and hydrophilic diluent (lactose) as well as using hydrophilic diluent (lactose) of various sizes were prepared using the granulation liquid or the solvent as indicated in the following list and table.

### Example 5: glimepiride : arginine = 1 : 0.5 + lactose

### Example 6: glimepiride : arginine = 1 : 25 + lactose

### Example 7: glimepiride : arginine = 1 : 2.5 - sample not containing lactose

### Example 8: glimepiride : arginine = 1 : 2.5 - sample using lactose of different particle size

| **Composition\ Process** | **Example 5** | **Example 6** | **Example 7** | **Example 8 **** |
|---|---|---|---|---|
| | granulation | granulation | * | granulation |
| **glimepiride** | 4 mg | 4 mg | 4 mg | 4 mg |
| **arginine** | 2 mg | 100 mg | 10 mg | 10 mg |
| **lactose** | 300 mg | 300 mg | - | 300 mg |
| **water** | 500 mg | 500 mg | 500 mg | 500 mg |
| **ethanol** | 500 mg | 500 mg | 500 mg | 500 mg |

| | | | | |
|---|---|---|---|---|
| * Example 7: arginine was dissolved in mixture ethanol / water, active ingredient glimepiride was added into solution. After the dissolution of glimepiride, solvent was evaporated in vaccum at 25 °C. ** Example 8: Different grade of lactose with bigger particles was used (d_{0.9} > 150µm) compared to lactose of Example 3 having smaller particle size of d_{0.9} < 150µm. | | | | |

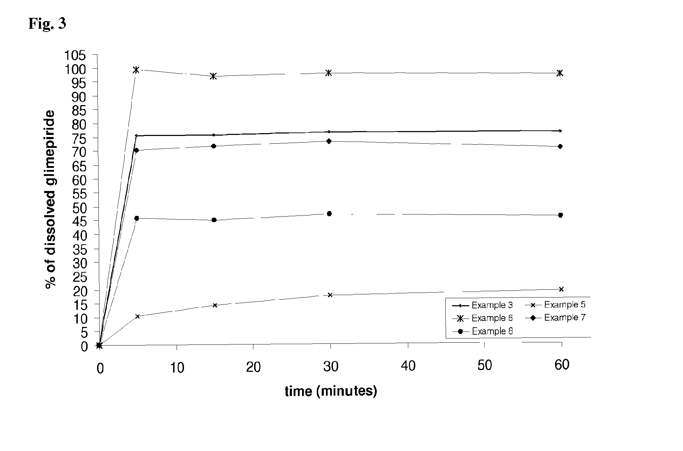

The results are shown in Fig. 3.

### Conclusions:

- the ratio of basic agent (arginine) : API (glimepiride) below 1 : 1, will not give satisfactory results, solubility of glimepiride is decreased - see Example 5
- increased weight ratio of basic agent (arginine) : API (glimepiride) of 25 : 1 is extremely effective - see Example 6
- the hydrophilic diluent (lactose) acts mainly as a carrier; it is not necessary for providing good solubility but hydrophilic diluent (lactose) further enhances solubility of glimepiride - see comparison between Example 3 and Example 7
- the size of particles of lactose has a remarkable influence on the solubility of the glimepiride. Smaller particles mean bigger area surface, faster wettability and enhanced dissolution. See comparison between Example 3 and Example 8. The significant size limit of hydrophilic diluent (lactose) particles is d_{0.9} < 150 µm.

### Example 9

In the following possible compositions of final dosage form are given.

### COMPOSITION 1

| | | **Per tablet (mg)** | |
|---|---|---|---|
| | **GRANULATE 1** | | |
| **1** | Glimepiride | 4,00 | Active pharm. ingredient |
| **2** | Arginine | 10,00 | Basic agent |
| **3** | Hydroxypropyl cellulose | 10,00 | Binder |
| **4** | Lactose | 151,00 | Soluble filler |
| **5** | Sodium croscarmellose | 5,00 | Disintegrant |
| **6*** | Demi water | 500,00 | Solvent, granulation lilquid |
| **7*** | Ethanol 96% | 500,00 | Solvent, granulation lilquid |

| | **GRANULATE 2** | | |
|---|---|---|---|
| **1** | Pioglitazone hydrochloride | 33,00 | Active pharm. ingredient |
| **2** | Povidone | 5,00 | Binder |
| **3** | Lactose | 80,00 | Soluble filler |
| **4** | Sodium croscarmellose | 2,00 | Disintegrant |
| **5*** | Demi water | 40,00 | Granulation liquid |

| | | **Per tablet (mg)** | |
|---|---|---|---|
| **1** | Dry granulate 1 | 180,00 | |
| **2** | Dry granulate 2 | 120,00 | |
| | | | Filler, |
| **3** | Microcrystalline cellulose, | 62,00 | separating agent |
| **4** | Sodium croscarmellose | 4,00 | Disintegrant |
| **5** | Magnesium stearate | 3,00 | Lubricant |
| | **tablet mass =** | **370,00** | |
| | Not present in tablet - | | |
| * | removed during processing | | |

### Technological procedure:

Glimepiride, arginine and hydroxypropyl cellulose are dissolved in water-ethanol mixture. Thus prepared granulation liquid is sprayed onto lactose and sodium corscarmellose mixture in fluid bed granulator. Dry granulate is labeled "Dry granulate 1".
Povidone is dissolved in demi water and sprayed onto pioglitazone hydrochloride, lactose and sodium corscarmellose mixture in fluid bed granulator. Dry granulate is labeled "Dry granulate 2". Dry granulate 1 and 2, microcrystalline cellulose and sodium croscaremllose are mixed in double cone mixer, finally magnesium stearate is added and mixed for a short period of time. Thus prepared granulate is compressed in rotary tabletting machine to obtain tablets with tablet mass 370 mg.

### COMPOSITION 2

| | | **Per tablet (mg)** | |
|---|---|---|---|
| | **GLIMEPIRIDE LAYER** | | |
| **1** | Glimepiride | 4,00 | Active pharm. ingredient |
| **2** | Arginine | 10,00 | Basic agent |
| **3** | Hydroxypropyl cellulose | 4,50 | Binder |
| **4** | Lactose | 125,50 | Soluble filler |
| **5** | Sodium croscarmellose | 3,00 | Disintegrant (intra-granular) |
| **6** | Sodium croscarmellose | 2,00 | Disintegrant (extra-granular) |
| **7** | Magnesium stearate | 1,00 | Lubricant |
| **8*** | Demi water | 400,00 | Solvent, granulation lilquid |
| **9*** | Ethanol 96% | 400,00 | Solvent, granulation lilquid |

| | **ROSUVASTATIN LAYER** | | |
|---|---|---|---|
| **1** | Rosuvastatin calcium | 41,55 | Active pharm. ingredient |
| **2** | Lactose anhydrous | 46,45 | Filler |
| **3** | Silicified microcrystalline | 40,00 | Filler, binder |
| | cellulose | | |
| **4** | Corn starch | 20,00 | Disintegrant |
| **5** | Sodium stearyl fumarate | 2,00 | Lubricant |

| | | **Per tablet (mg)** | |
|---|---|---|---|
| **1** | Glimpeiride layer | 150,00 | |
| **2** | Rosuvastatin layer | 150,00 | |
| | **tablet mass =** | **300,00** | |
| * | Not present in tablet - removed | | |
| | during processing | | |

### Technological procedure:

Glimepiride, arginine and hydroxypropyl cellulose are dissolved in water-ethanol mixture. Thus prepared granulation liquid is sprayed onto lactose and sodium corscarmellose mixture in fluid bed granulator. Extragranular sodium croscarmellose is added and mixed. Finally magnesium stearate is added and mixed for a short period of time. Final blend for glimepiride layer is thus obtained.

Rosuvastatin calcium, lactose anhydrous, silicified microcrystalline cellulose and corn starch are mixed in convection mixer, sieved and mixed again. Finally sodium stearyl fumarate is added and mixed for a short period of time. Final blend for rosuvastatin layer is obtained.

Thus prepared granulates are compressed in a rotary tabletting machine using a bi-layer mode to obtain bi-layer tablets with tablet mass 300 mg.

## Claims

1. A pharmaceutical composition comprising at least one sulphonylurea-based active pharmaceutical ingredient, wherein the at least one sulphonylurea-based active pharmaceutical ingredient is mixed with a basic agent and the weight ratio of the basic agent to the at least one sulphonylurea-based active pharmaceutical ingredient is from above 1:1 to 50:1.

2. A pharmaceutical composition comprising at least one sulphonylurea-based active pharmaceutical ingredient, wherein the at least one sulphonylurea-based active pharmaceutical ingredient is intimately mixed with a basic agent at a weight ratio of the basic agent to the at least one sulphonylurea-based active pharmaceutical ingredient being higher than 1:1, wherein the intimate mixture is obtained by partially or fully dissolving both substances in a solvent and by subsequently at least partially precipitating both substances.

3. The pharmaceutical composition according to claim 1 or 2, wherein the intimately mixed basic agent and the at least one sulphonylurea-based active pharmaceutical ingredient are deposited on a hydrophilic diluent and/or homogenously mixed with the hydrophilic diluent.

4. A pharmaceutical composition comprising at least one sulphonylurea-based active pharmaceutical ingredient, further comprising a basic agent at a weight ratio of the basic agent to the at least one sulphonylurea-based active pharmaceutical ingredient being higher than 1:1, and wherein the basic agent and the at least one sulphonylurea-based active pharmaceutical ingredient are deposited on hydrophilic diluent particles and/or mixed with hydrophilic diluent particles, wherein the particle size of the particulate hydrophilic diluent is defined by d_{0.9} < 150µm.

5. The pharmaceutical composition according to any one of the preceding claims, wherein the composition further comprises at least one polymer.

6. The pharmaceutical composition according to any one of the preceding claims, wherein the composition further comprises at least one other active pharmaceutical ingredient selected from the group consisting of thiazolidinone class compounds, biguanide class compounds, statin class compounds, fibrate class compounds, acarbose, miglitol, voglibose, orlistat, sibutramine, rimonabant, exenatide and pramlintide, preferably selected from the group consisting of biguanide class compounds and thiazolidinone class compounds, more preferably thiazolidinone class compounds, particularly pioglitazone or rosiglitazone.

7. The pharmaceutical composition according to any one of the preceding claims, wherein the composition is formulated in a dosage form, preferably a tablet.

8. The pharmaceutical composition according to any one of the preceding claims, wherein the at least one sulphonylurea-based active pharmaceutical ingredient is selected from the group consisting of acetohexamide, chlorpropamide, tolbutamide, tolazamide, glipizide, gliclazide, glibenclamide, gliquidone, glyclopyramide, glimepiride, gliquidone, glixosepid, glimidine, glypinamide, glyhexamide, glibuzole and glybuthiazole, preferably from the group of glipizide, gliclazide, glibenclamide and glimepiride, most preferably is glimepiride.

9. The pharmaceutical composition according to any one of the preceding claims, wherein the basic agent has a pKa over 9 and a solubility in water of at least 10 mg/mL.

10. A process for preparing a pharmaceutical composition comprising at least one sulphonylurea-based active pharmaceutical ingredient, wherein the at least one sulphonylurea-based active pharmaceutical ingredient is admixed with a basic agent, optionally in the presence of a granulation liquid or a solvent, wherein the weight ratio of the basic agent and the at least one sulphonylurea-based active pharmaceutical ingredient is from above 1:1 to 50:1.

11. A process for preparing a pharmaceutical composition comprising at least one sulphonylurea-based active pharmaceutical ingredient, wherein the at least one sulphonylurea-based active pharmaceutical ingredient and a basic agent are partially or fully dissolved in a granulation liquid or a solvent, wherein the weight ratio of the basic agent and the at least one sulphonylurea-based active pharmaceutical ingredient is above 1:1, and at least partly precipitated.

12. The process according to claim 10 or 11, wherein the at least one sulphonylurea-based active pharmaceutical ingredient and a basic agent are deposited on hydrophilic diluent and/or homogenously mixed with the hydrophilic diluent;
or wherein the at least one sulphonylurea-based active pharmaceutical ingredient and a basic agent are coated on pharmaceutical core.

13. A process for preparing a pharmaceutical composition comprising at least one sulphonylurea-based active pharmaceutical ingredient, wherein the at least one sulphonylurea-based active pharmaceutical ingredient and a basic agent are partially or fully dissolved in a granulation liquid or a solvent, and subsequently the obtained partial or complete solution is sprayed onto hydrophilic particles and/or intimately mixed with hydrophilic particles, the hydrophilic particles being selected from the group consisting of modified or unmodified monosaccharides, straight or branched oligosaccharides, and straight or branched polysaccharides, wherein the particle size of the particulate hydrophilic diluent is defined by d_{0.9} < 150µm.

14. The process according to any one of claims 10 to 13, wherein the weight ratio of the basic agent and the at least one sulphonylurea-based active pharmaceutical ingredient is from 1.1:1 to 50:1, preferably from 1.5:1 to 25:1, more preferably from 2:1 to 15:1, particularly from 3:1 to 10:1.

15. The process according to any one of claims 10 to 14, wherein the granulation liquid or the solvent is water, at least one organic solvent or mixtures thereof.

16. The process according to any one of claims 10 to 15, wherein a polymer is further added to the composition.

17. Use of pharmaceutical composition according to any one of claims 1 to 9 for preparing a medicament.
